# FASCICULE DE BREVET EUROPEEN

(11) **EP 3 986 486 B1**
(45) Date de publication et mention de la délivrance du brevet: **18.11.2026**
(21) Numéro de dépôt: 19848793.6
(22) Date de dépôt: 19.12.2019
(51) Int. Cl.: A61L 9/12, A61L 9/14, A61L 9/04, A01M 1/02, A61L 9/03, A01M 7/00, A01M 29/12, A01M 1/20

(54) **ENSEMBLE AMOVIBLE POUR UN APPAREIL DIFFUSEUR**
ABNEHMBARE ANORDNUNG FÜR EINE STREUVORRICHTUNG
REMOVABLE ASSEMBLY FOR A DIFFUSING APPARATUS

(30) Priorité: 18.06.2019 WO PCT/FR2019/051490
(43) Date de publication de la demande: 27.04.2022
(73) Titulaire: CAELIMP, 31670 Labege (FR)
(72) Inventeur: PEREZ, Yoann, 65410 Sarrancolin (FR); RIVIERE, Philippe, 31500 Toulouse (FR); PICHON, Philippe, 31800 VILLENEUVE-DE-RIVIERE (FR)
(74) Mandataire: Loyer & Abello
(86) Numéro de dépôt international: PCT/FR2019/053207
(87) Numéro de publication internationale: WO 2020/254733

(56) Documents cités:
- WO-A1-2008/124959
- US-A1- 2014 238 422
- US-A1- 2017 072 085
- US-A1- 2018 098 573
- US-B2- 8 483 553
- US-B2- 9 616 149

## Description

### Domaine technique

L'invention se rapporte au domaine des appareils diffuseurs destinés à diffuser une substance à l'état vapeur dans l'air ambiant, la substance étant un liquide comportant au moins un composé sélectionné parmi les molécules sémiochimiques, les phéromones, les allomones, les kairomones, les synomones et les parfums d'origine naturelle ou synthétique. En particulier, l'invention se rapporte à un ensemble amovible pouvant être utilisé dans un tel appareil diffuseur.

### Arrière-plan technologique

Des composés tels que les molécules sémiochimiques, les phéromones, les allomones, les kairomones, les synomones et les parfums d'origine naturelle ou synthétique peuvent être utilisés dans de nombreuses applications. Dans des applications agrochimiques, des substances sémiochimiques sont par exemple utilisées pour éloigner ou contrôler des populations d'animaux ravageurs.

US-A-20180000977A1 décrit un appareil vaporisateur pour étendre la portée et l'efficacité des odeurs. L'appareil comprend un récipient amovible contenant une odeur.

US-A-2140516 décrit un générateur de vapeur électrique générant de la vapeur d'eau de manière instantanée, la vapeur pouvant contenir un médicament ou un fumigant. Les documents US8483553B2 et US2017072085A1 décrivent aussi des diffuseurs connus.

### Résumé

Certains aspects de l'invention partent de l'idée de proposer un ensemble amovible pour un appareil diffuseur, l'appareil diffuseur étant destiné à diffuser une substance à l'état vapeur dans l'air ambiant permettant une consommation d'énergie faible pour assurer une grande durée d'autonomie.

Certains aspects de l'invention partent de l'idée de proposer un ensemble amovible pour un appareil diffuseur qui régule un débit de substance distribuée à travers un corps poreux par un simple contrôle de température.

Certains aspects de l'invention partent de l'idée de proposer un ensemble amovible pour un appareil diffuseur adapté particulièrement pour utiliser une substance de grande valeur avec une précision élevée sans pertes de substance.

Dans le cadre d'une substance onéreuse, par exemple la substance comprend une phéromone, se présentant sous forme liquide à température ambiante, il faut éviter d'en gaspiller. Ainsi dans ce cas figure, on désire amener jusqu'à une surface d'évaporation d'un organe distributeur poreux une quantité de liquide suffisamment faible pour que l'écoulement s'effectue sans formation de goutte.

L'écoulement du liquide à travers un corps poreux est gouverné à froid par la loi de Jurin et à chaud par la loi de Darcy.

La loi de Darcy s'énonce ainsi :$Q = KA \left(ΔH\right) / L ,$ où Q est le débit volumique, K est la conductivité hydraulique, A est la surface de la section étudiée du corps poreux, ΔH est la différence des hauteurs piézométriques en amont et en aval de l'échantillon de corps poreux et L est la longueur de l'échantillon du corps poreux. La conductivité hydraulique se calcule avec la formule :$K = kpg / μ ,$ où k est la perméabilité intrinsèque du corps poreux, ρ est la masse volumique de la substance liquide, g est l'accélération de pesanteur et µ est la viscosité de la substance liquide.

La loi de Jurin correspond à la formule :$h = \left(2γcos\left(θ\right)\right) / \left(rρg\right) ,$ où h est la hauteur du liquide, γ est la tension superficielle du liquide, θ est l'angle de contact entre le liquide et la paroi des micro-canalisations, ρ est la masse volumique du liquide, r est le rayon des micro-canalisations, et g est la constante de la gravité.

On appellera « ensemble amovible » un ensemble comportant notamment des éléments consommables, notamment la substance liquide à diffuser, et pouvant être déplacé, inséré ou retiré d'une seule pièce d'un appareil diffuseur, par opposition à une partie fixe de l'appareil diffuseur qui comporte notamment des organes dont la durée de vie est plus grande.

La conception d'un tel ensemble amovible pour un appareil diffuseur peut être faite de différentes manières, à partir du récipient de stockage de la substance liquide et en intégrant avec celui-ci un nombre plus ou moins élevé d'éléments de l'appareil diffuseur, notamment des éléments dont la fonction est de distribuer la substance liquide en dehors du récipient de stockage et/ou des éléments dont la fonction est de générer et/ou diriger un flux d'air destiné à évaporer la substance liquide. Le choix d'intégrer un élément de l'appareil diffuseur à la partie amovible plutôt qu'à la partie fixe peut être fondé sur plusieurs considérations. Une première considération est l'intégration mécanique de l'élément avec le récipient de stockage, par exemple dans l'objectif de limiter l'encombrement de l'appareil ou le coût de fabrication. Par exemple l'intégration à l'ensemble amovible de certains éléments qui concourent à générer ou guider le flux d'air peut servir cet objectif. Une deuxième considération est la relation fonctionnelle entre l'élément et la substance liquide à distribuer, par exemple dans l'objectif de favoriser la compatibilité de l'appareil diffuseur avec plusieurs substances différentes sans risque de pollution ou autrement dit sans mélange non désiré. Par exemple l'intégration à l'ensemble amovible de certains éléments qui concourent à conduire la substance liquide peut servir cet objectif.

La solution au problème technique est apportée par l'objet de la revendication indépendante 1, définissant en soi l'invention. Des modes de réalisation préférés sont décrits dans les revendications dépendantes.

Selon un mode de réalisation, la présente invention propose un ensemble amovible pour un appareil diffuseur, l'appareil diffuseur étant destiné à diffuser une substance à l'état vapeur dans l'air ambiant, l'ensemble amovible comprenant un récipient de stockage comprenant un orifice et contenant une substance liquide.

Un tel ensemble amovible peut être conçu pour diffuser différentes substances.

Selon un mode de réalisation, ladite substance liquide comporte au moins un composé sélectionné parmi les molécules sémiochimiques, les phéromones, les allomones, les kairomones, les synomones d'origine naturelle ou synthétique.

Selon un mode de réalisation, la substance est une solution renfermant au moins une phéromone sexuelle ou non, une allomone, une synomone ou une kairomone destinée à provoquer une réponse positive ou négative relativement à l'espèce visée, dont le résultat comportemental peut être une confusion sexuelle, une confusion d'autre nature, une attraction sexuelle, une attraction d'autre nature, une répulsion de toute nature, chez les arthropodes, dont les arachnides, ou dont les hexapodes, parmi lesquels notamment les insectes, dont les insectes nuisibles.

Selon un mode de réalisation, la substance est une solution renfermant au moins une phéromone ou une phéromone sexuelle, une allomone, une synomone ou une kairomone destinée à provoquer une réponse positive ou négative relativement à l'espèce visée, dont le résultat comportemental peut être notamment un apaisement, une relaxation, une euphorisation ou une intimidation chez les classes mammalia et aves.

Selon un mode de réalisation, la substance comprend un solvant choisi parmi le myristate d'isopropyle, le dipropylène glycol, Éther de dipropylène glycol monométhylique, et un hydrocarbure isoparaffinique, par exemple une isoparaffine L ou P ou N ou V.

Selon un mode de réalisation, la substance est une solution comportant au moins un composé pris dans le groupe formé par les agents odorifères utilisables pour l'homme ou l'animal, les substances sémiochimiques, les agents cosmétiques, les huiles essentielles, les parfums et les agents phytosanitaires et agricoles.

Selon un mode de réalisation, les agents odorifères utilisables pour l'animal sont choisis parmi les acides gras ou la forme estérifiée desdits acides gras telle que l'oléate de méthyle, le palmitate de méthyle, l'azélate de diméthyle, et le pimelate de diméthyle.

Selon un mode de réalisation, la substance liquide présente une viscosité supérieure à 1 cPa.s à 25°C, par exemple supérieure à 8 cPa.s à 25°C, et inférieure à 1 cPa.s à 60°C.

Selon un mode de réalisation, la substance présente une température d'ébullition comprise entre 30°C et 400°C à la pression atmosphérique.

Selon un mode de réalisation, ledit récipient de stockage contient en outre un organe de rétention alvéolaire interne imprégné de ladite substance liquide.

Selon un mode de réalisation l'organe de rétention alvéolaire comprend un matériau choisi parmi un feutre, par exemple feutre en laine, et une mousse de mélamine.

Selon un mode de réalisation, une pluralité d'organes de rétention alvéolaires sont en contact et disposés dans le récipient de stockage.

Selon des modes de réalisation, les organes de rétention alvéolaires peuvent posséder des raideurs différentes et/ou des duretés différentes et/ou des densités différentes.

Selon un mode de réalisation, ledit organe de rétention alvéolaire interne est logé au moins dans une zone d'embouchure dudit récipient de stockage adjacente à l'orifice, ledit organe de rétention alvéolaire étant en retrait dudit orifice.

Selon un mode de réalisation, l'organe de rétention alvéolaire couvre la section complète de la zone d'embouchure adjacente à l'orifice du récipient de stockage.

Grâce à un tel organe de rétention alvéolaire, la substance liquide peut être retenue de manière fiable dans le récipient par capillarité sans risquer de s'écouler de manière incontrôlée tant que le récipient de stockage n'est pas connecté à l'organe distributeur sous la forme d'un corps poreux.

Selon un mode de réalisation, une pluralité d'organes de rétentions alvéolaires internes sont disposés dans ledit récipient de stockage, un premier organe de rétention alvéolaire interne situé dans ladite zone d'embouchure dudit récipient de stockage étant plus raide qu'un deuxième organe de rétention alvéolaire interne disposé à distance de la zone d'embouchure.

Selon un mode de réalisation, un moyen de maintien dudit organe de rétention alvéolaire interne est situé dans ledit récipient et s'étend d'une extrémité dudit récipient de stockage opposée audit orifice pour maintenir ledit organe de rétention alvéolaire afin de le maintenir en place dans ladite zone d'embouchure.

Selon un mode de réalisation, ledit moyen de maintien de l'organe de rétention alvéolaire interne comporte une tige fixée à l'extrémité dudit récipient de stockage opposée audit orifice. Par exemple, une tige pleine avec une extrémité en forme de croix, ladite extrémité étant en contact avec l'organe de rétention alvéolaire.

Selon un mode de réalisation, le récipient de stockage comprend une lèvre de maintien agencée autour de l'orifice et saillante vers l'intérieur. Ainsi, le pourtour interne de la lèvre de maintien possède une ouverture au dimensions inférieures à l'orifice. Ces dimensions permettent de contenir l'organe de rétention alvéolaire interne en retrait dans le récipient de stockage

L'ensemble amovible peut comporter un ou plusieurs éléments qui concourent à distribuer la substance liquide en dehors du récipient de stockage. Selon un mode de réalisation, ledit ensemble amovible comprend un organe distributeur sous la forme d'un corps poreux présentant une surface d'évaporation située à l'extérieur dudit récipient de stockage pour évaporer la substance dans l'air ambiant. En d'autres termes, le corps poreux comprend des pores qui constituent des micro-canalisations débouchant sur la surface d'évaporation.

On appellera « micro-canalisation » une canalisation, dont la section droite a une aire comprise entre 10⁻⁴ et 10⁶ µm². Selon un mode de réalisation, lesdits pores présentent un diamètre compris entre 0.01 et 10 µm.

Selon un mode de réalisation le corps poreux comporte une mèche en bois, en textile, en céramique ou en polymère.

Selon un mode de réalisation, le corps poreux présente une forme de cylindre.

Selon un mode de réalisation, le corps poreux présente une porosité dans une partie intérieure du corps poreux plus faible qu'une porosité dans une partie extérieure du corps poreux entourant la partie intérieure. Cela permet de contrôler le débit d'écoulement dans le corps poreux avec la faible porosité et augmenter les échanges avec l'air avec la forte porosité de surface.

Un tel organe distributeur peut être relié de différentes manières, directement ou indirectement, à l'orifice du récipient.

Selon un mode de réalisation, une liaison entre le récipient de stockage et son organe distributeur associé est assurée au moyen d'une tuyauterie.

Selon un mode de réalisation convenant pour un appareil diffuseur dont la partie fixe comprend l'organe distributeur, ledit organe distributeur comprend une aiguille creuse configurée pour percer un opercule et/ou pour déplacer une membrane faisant clapet dudit récipient de stockage et amener la substance contenue dans le récipient de stockage jusqu'à la surface d'évaporation, ledit opercule ou ladite membrane étant situé en sortie dudit orifice et étant destinés à être perforés.

Selon un mode de réalisation, l'aiguille est disposée à une des extrémités du corps poreux. Une telle aiguille peut aussi être employée en combinaison avec un bouchon perforable « auto-cicatrisant » logé dans l'orifice du récipient de stockage, c'est-à-dire une masse de matière élastique qui referme élastiquement la perforation réalisée par l'aiguille, de sorte qu'aucun écoulement n'a lieu après le retrait de celle-ci.

Selon un mode de réalisation, ledit opercule est réalisé en plastomère ou un film métallique.

Selon un mode de réalisation, ledit organe distributeur est positionné en sortie dudit orifice, et le corps poreux est assemblé de manière étanche au récipient et présente une portion d'extrémité engagée dans l'orifice pour entrer en contact avec ledit organe de rétention alvéolaire.

Grâce à ces caractéristiques, la distribution de la substance liquide entre le récipient et la surface d'évaporation peut être réalisée par contact direct du corps poreux avec l'organe de rétention, ce contact générant une traction capillaire.

Selon un mode de réalisation, la portion d'extrémité comprend un ergot agencé sur une partie supérieure dudit corps poreux et s'étendant selon un axe longitudinal du corps poreux et configuré pour recevoir la substance par contact avec l'organe de rétention alvéolaire.

Selon un mode de réalisation, ledit ensemble amovible comporte au moins un organe de chauffage permettant de chauffer ledit corps poreux et un contact électrique associé à l'organe de chauffage et destiné à réaliser une liaison électrique avec une partie fixe de l'appareil diffuseur.

Selon des modes de réalisation, l'ensemble amovible ou la partie fixe comprend au moins un capteur de température mesurant la température de l'organe distributeur et/ou la température du flux d'air. Selon un mode de réalisation, l'organe distributeur est équipé d'un capteur de température, par exemple au niveau d'une extrémité libre.

Le capteur de température peut être par exemple un thermomètre à dilatation ou une thermistance.

Selon un mode de réalisation l'organe de chauffage et le récipient de stockage sont disposés de part et d'autre de l'organe distributeur au sein de l'ensemble amovible.

Selon un mode de réalisation, l'organe de chauffage est placé directement sur une surface du corps poreux.

Selon un mode de réalisation, le corps poreux présente au moins un évidement logeant au moins une partie de l'organe de chauffage.

Selon un mode de réalisation, l'organe de chauffage est configuré pour réguler un débit de la substance à travers l'organe distributeur en modifiant une viscosité de la substance sans atteindre le point d'ébullition de la substance.

Selon un mode de réalisation, une température de consigne est définie en fonction de la substance.

L'écoulement de la substance liquide peut être contrôlé de différentes manières. L'organe distributeur poreux permet notamment de mettre en œuvre un contrôle de l'écoulement de la substance liquide qui soit basé essentiellement sur la température du corps poreux.

Pour cela, on désire qu'à température ambiante, sans chauffage de l'organe distributeur poreux, la conductivité hydraulique K soit trop faible pour qu'un écoulement ait lieu, c'est-à-dire qu'on se trouve dans une situation dite « capillaire ».

En revanche à chaud, il est souhaitable que l'écoulement soit suffisant pour produire un étalement de la substance liquide sur la surface extérieure du corps poreux, qui forme la surface d'évaporation et que le liquide adhère à cette surface. La couche de liquide adhérant à la surface modifie la différence des hauteurs piézométriques ΔH et aboutit ainsi à couper le débit de l'écoulement du fait que la conductivité hydraulique K a atteint une valeur maximale. En d'autres termes l'écoulement à chaud, c'est-à-dire à la température imposée par l'organe de chauffage, ne se produit que dans la mesure où la couche de liquide adhérant à la surface est consommée par l'évaporation et aucune goutte ne se détache de la surface du corps poreux.

Les paramètres les plus importants sont donc la viscosité du fluide, sa tension superficielle et la température du corps poreux.

Dans un exemple de réalisation, cosθ est positif c'est-à-dire que la substance est mouillante sur l'organe distributeur, par exemple en céramique, la masse volumique du liquide est comprise entre 0,6 et 1g/cm³, et le rayon des micro-canalisations est compris en 5 nm et 1 µm.

A froid, c'est à dire à une température ambiante inférieure à un seuil de température, la surface de liquide évaporable est donc très faible : il s'agit de la somme des sections des micro-canalisations débouchant sur la surface d'évaporation. On peut donc obtenir un état dans lequel la substance liquide est en retrait dans le corps poreux sans possibilité d'écoulement. Dans cet état, l'évaporation à froid dépend de la volatilité du liquide et peut être sensiblement nulle si cette volatilité est suffisamment faible. Le seuil de température dépend à la fois des propriétés physiques du corps poreux (porosité, section des pores), des propriétés physiques du liquide (viscosité, tension superficielle) et des forces motrices susceptibles de générer un écoulement (gravité, pression).

La baisse de la viscosité dynamique de la substance avec la chaleur fournie par un organe de chauffage permet au fluide de circuler au sein de l'organe distributeur selon la loi de Darcy puis de s'étaler en surface dudit organe distributeur. Sans apport de chaleur, la circulation est figée car la somme des adhérences au sein de l'organe distributeur suit la loi de Jurin. En d'autres termes, l'écoulement est permis à travers l'organe distributeur à chaud, en raison de la baisse de viscosité dynamique induite par la montée en température, mais retenu en surface par la force d'adhérence entre le fluide et la surface de l'organe distributeur, en conséquence de la mouillabilité du fluide sur la surface d'évaporation. A température ambiante, la force d'adhérence entre le fluide et la surface des micro-canalisations à l'intérieur du corps poreux est telle que le liquide demeure confiné au sein de l'organe distributeur.

Lors de l'écoulement, il faut plus d'énergie pour former une goutte qui se détachera que pour maintenir la substance liquide au sein de l'organe distributeur et du récipient de stockage. Cela tient à deux conditions :
1. la viscosité dynamique de la substance ne doit pas être trop faible dans la plage de température pouvant être atteinte à l'aide de l'organe de chauffage, et
2. le liquide en sortie de réservoir doit être en équilibre avec la pression atmosphérique, ce qui peut être mis en œuvre de plusieurs manières. Par exemple, la partie dépourvue de liquide dans le récipient de stockage est en dépression. Alternativement, un système de gestion de pression de la partie dépourvue de liquide du récipient assure cet équilibre.

Selon un mode de réalisation, ladite substance présente une viscosité variable en fonction de la température, ladite viscosité et la tension superficielle de la substance liquide étant telles que lorsque l'accélération de pesanteur s'exerce depuis l'orifice en direction du corps poreux, ladite substance ne peut pas s'écouler à travers ledit corps poreux à une température ambiante inférieure à une première température, la première température étant supérieure à 0°C et ladite substance pouvant s'écouler à travers ledit corps poreux à une deuxième température supérieure à ladite première température.

La première température peut être fixée dans différentes plages. Si l'appareil diffuseur est destiné à être utilisé en plein air, la première température sera notamment choisie en fonction des données climatiques locales. Selon des modes de réalisation, la première température est par exemple comprise entre 1°C et 50°C, ou entre 5°C et 40°C ou entre 10°C et 35°C, ou encore entre 15°C et 25°C.

L'ensemble amovible peut comporter des éléments qui concourent à générer et/ou diriger un flux d'air destiné à évaporer la substance liquide. Selon un mode de réalisation, ledit ensemble amovible comprend un guide flux d'air situé en périphérie dudit orifice.

Selon un mode de réalisation, ledit guide flux d'air est destiné à guider un flux d'air dans une direction tangentielle à la surface d'évaporation ou à l'emplacement destiné à la surface d'évaporation, et ledit ensemble amovible comprend des éléments d'étanchéités pour réaliser des liaisons étanches entre le récipient et ledit guide flux et entre le guide flux et ledit corps poreux.

Selon un mode de réalisation, l'ensemble amovible comprend une veine aéraulique tubulaire s'étendant autour du corps poreux ou de l'emplacement destiné au corps poreux, ladite veine permettant de guider le flux d'air sur ledit corps poreux.

Selon un mode de réalisation, ledit récipient de stockage présente une forme allongée selon une direction axiale, ledit orifice étant situé à une extrémité axiale du récipient de stockage, la surface d'évaporation dudit corps poreux étant parallèle à la direction axiale, ledit guide flux comportant une pluralité d'ailettes parallèles à la direction axiale et disposées autour dudit récipient.

Selon un mode de réalisation, ledit ensemble amovible comprend tout ou partie d'une chambre de brassage d'air, ladite chambre de brassage d'air s'étendant tout autour dudit récipient. Dans un mode de réalisation, l'ensemble amovible comprend une paroi supérieure de la chambre de brassage d'air, la paroi supérieure s'étendant tout autour du récipient de stockage.

Selon un mode de réalisation, ladite chambre de brassage d'air comprend une ouverture permettant la mise en relation avec un ventilateur.

Selon un mode de réalisation, ladite chambre de brassage d'air comprend un ventilateur et une jonction électrique, ledit ventilateur étant positionné de manière à générer un flux d'air au sein de ladite chambre de brassage d'air, ladite jonction électrique étant destinée à s'associer à un connecteur électrique au sein d'une partie fixe du dispositif de diffusion pour alimenter le ventilateur. Par exemple le débit d'air du ventilateur peut être compris entre 0,2 et 60 m³/h.

Selon un mode de réalisation, ledit guide flux présente une pluralité de canaux débouchant sur la chambre de brassage et orientés dans la direction tangentielle à la surface d'évaporation ou à l'emplacement destiné à la surface d'évaporation.

Selon un mode de réalisation, ledit ensemble amovible comprend une collerette faisant saillie autour du récipient de stockage et adaptée pour recouvrir une ouverture d'une partie fixe de l'appareil diffuseur, ladite ouverture étant destinée à l'insertion dudit ensemble amovible dans la partie fixe de l'appareil diffuseur.

Selon un mode de réalisation, l'ensemble amovible comprend un organe régulateur d'un débit d'air, configuré pour contrôler le ventilateur afin de réguler un débit d'air dans ladite chambre d'aération et/ou ledit guide flux d'air.

Le récipient de stockage peut être réalisé de différentes manières.

Selon un mode de réalisation, ledit récipient de stockage comporte un évent de pressurisation mettant en communication l'espace intérieur du récipient de stockage avec l'air ambiant.

Selon un mode de réalisation, l'ensemble amovible comporte un récipient de stockage extérieur et un récipient de stockage intérieur logé dans le récipient extérieur, ledit récipient de stockage intérieur étant en liaison avec l'organe distributeur à travers l'orifice et présentant un évent relié à l'atmosphère à une extrémité opposée à l'orifice, une ouverture de communication entre le récipient de stockage extérieur et le récipient de stockage intérieur étant agencé dans la zone d'embouchure à proximité de l'orifice, le récipient de stockage extérieur ne présentant pas d'autre ouverture que l'ouverture de communication. De préférence, un ou plusieurs organes de rétention alvéolaires sont disposés dans le récipient de stockage intérieur et/ou le récipient de stockage extérieur.

Selon un mode de réalisation convenant pour un appareil diffuseur dont la partie fixe comprend l'organe distributeur, l'ensemble amovible comporte un bouchon, ledit bouchon étant disposé pour boucher ledit orifice de manière étanche durant le transport dudit ensemble amovible. Par exemple, hors utilisation dans l'appareil, c'est -à-dire avant que le récipient ne soit mis en connexion avec l'organe distributeur ou après avoir été déconnecté de l'organe distributeur, un tel récipient de stockage peut être muni d'un bouchon agencé sur l'orifice de vidange.

Selon un mode de réalisation, l'ensemble amovible comprend un joint d'étanchéité agencé autour de l'orifice, de manière à assurer une liaison étanche entre le récipient de stockage et l'organe distributeur.

Selon un mode de réalisation, l'ensemble amovible comprend un moyen de maintien mécanique permettant de stabiliser l'ensemble amovible dans ladite partie fixe de l'appareil diffuseur. Par exemple, l'ensemble amovible est monté dans ladite partie fixe dudit appareil de diffusion par vissage ou encliquetage.

Selon un mode de réalisation, le récipient de stockage ne présente pas d'autre ouverture que l'orifice, ledit récipient de stockage contenant, outre la substance liquide, une phase gazeuse occupant au moins 20% du volume du récipient de stockage.

Selon un mode de réalisation, l'ensemble amovible comprend un moyen mécanique de détrompage pour faciliter l'insertion de l'ensemble amovible dans sa position de fonctionnement dans la partie fixe de l'appareil diffuseur.

Selon un mode de réalisation, ledit corps poreux est configuré pour établir une jonction thermique avec des éléments chauffants de l'appareil de diffusion.

Selon un mode de réalisation, ledit ensemble amovible est agencé en jonction de contact entre l'organe de rétention alvéolaire et l'organe distributeur lors de son insertion dans la partie fixe du dispositif de diffusion.

Selon un mode de réalisation, l'orifice du récipient relié à l'organe distributeur est orienté vers le bas, c'est-à-dire dans la direction de l'accélération de la pesanteur, lorsque l'appareil est dans une position d'utilisation.

La présente invention a également pour objet un appareil de diffusion comprenant ledit ensemble amovible selon un des modes de réalisation précités et une partie fixe comportant un boîtier qui définit un logement dans lequel ledit ensemble amovible est inséré dans une position de fonctionnement permettant la diffusion de la substance à l'état vapeur dans l'air ambiant.

Selon des modes de réalisation, ladite partie fixe comporte un boîtier externe et essentiellement tous les éléments de l'appareil diffuseur qui n'ont pas été intégrés à l"ensemble amovible, à savoir, seul ou en combinaison de manière non exhaustive: un guide flux, une veine aéraulique, une chambre ou partie de chambre de brassage, un ventilateur, un organe distributeur, un organe de chauffage, un module de communication, un dispositif de commande, par exemple sous la forme d'une carte électronique, un moyen de fixation pour fixer l'ensemble amovible dans le logement, par exemple par encliquetage ou vissage.

Un couvercle du boîtier peut être intégré à la partie fixe ou à l'ensemble amovible, par exemple sous la forme d'une collerette attachée au récipient de stockage.

Selon des modes de réalisation, la partie fixe ou ledit ensemble amovible comporte au moins un élément électronique de régulation configuré pour réguler l'organe de chauffage.

Selon un mode de réalisation, l'élément électronique de régulation comporte au moins une carte électronique et au moins une résistance électrique alimentée électriquement par la carte électronique. La résistance électrique peut être disposée sur ladite carte électronique, ou déportée de celle-ci.

Selon un mode de réalisation, l'élément électronique de régulation est configuré pour commander l'organe de chauffage en fonction d'une température de consigne dans l'organe distributeur.

Selon un mode de réalisation, l'ensemble amovible comprend un moyen pour identifier la substance stockée dans le récipient, par exemple un moyen numérique et électromagnétique tel qu'une puce RFID (de l'acronyme anglais « radio frequency identification ») ou un moyen analogique tel qu'un code barre ou autre marquage.

Selon un mode de réalisation, l'élément électronique de régulation est relié à un détecteur disposé dans la partie fixe et configuré pour détecter un marquage au niveau du récipient de stockage indicatif de la substance contenue dans le récipient et le dispositif de commande détermine en fonction dudit marquage, au moins un paramètre de fonctionnement du dispositif parmi la température de consigne, un débit d'air et des indications temporelles définissant un cycle arrêt/marche. De telles indications temporelles incluent par exemple des dates de début de cycle, dates de fin de cycle, durées de cycle, durée inter-cycles, etc.

Selon un mode de réalisation, l'élément électronique de régulation comporte une mémoire stockant une table de valeurs associant des substances à des températures de consigne.

Selon un mode de réalisation, la partie fixe comporte en outre un module de communication pour assurer une communication filaire ou non filaire avec un serveur de données, par exemple afin de modifier la table de valeurs ou mettre à jour le programme de régulation.

Selon un mode de réalisation, la partie fixe comporte des logements pour recevoir une pluralité de récipients de stockage contenant chacun une substance liquide.

### Brève description des figures

L'invention sera mieux comprise, et d'autres buts, détails, caractéristiques et avantages de celle-ci apparaîtront plus clairement au cours de la description suivante de plusieurs modes de réalisation particuliers de l'invention, donnés uniquement à titre illustratif et non limitatif, en référence aux dessins annexés.
La figue 1 représente une vue éclatée d'un appareil diffuseur pouvant être réalisé sous forme d'une partie fixe d'un ensemble amovible.
La figure 2 représente une vue éclatée en coupe de l'appareil diffuseur de la figure 1.
La figure 3 représente un récipient de stockage selon un premier mode de réalisation.
La figure 4 représente un récipient de stockage selon un deuxième mode de réalisation.
La figure 5 représente un récipient de stockage selon un troisième mode de réalisation à double récipient de stockage.
La figure 6 est une vue en perspective qui représente un récipient de stockage comprenant un bouchon selon un mode de réalisation.
La figure 7 représente un organe distributeur avec des organes de chauffage selon un mode de réalisation.
La figure 8 représente un ensemble amovible comprenant un récipient de stockage et un guide flux d'air selon un mode de réalisation.
La figure 9 est une vue en perspective qui représente un ensemble amovible comprenant un récipient de stockage, un guide flux d'air et un organe distributeur selon un mode de réalisation.
La figure 10 est une vue en coupe qui représente un ensemble amovible comprenant un récipient de stockage, le guide flux d'air et la veine aéraulique selon un mode de réalisation.
La figure 11 est une vue écorchée en perspective qui représente un ensemble amovible comprenant un récipient de stockage, une chambre de brassage d'air, un guide flux d'air, une veine aéraulique et un organe distributeur selon un mode de réalisation.
La figure 12 est une vue en perspective qui représente un ensemble amovible comprenant un récipient de stockage, une chambre de brassage d'air, un ventilateur, un guide flux d'air et une veine aéraulique selon un mode de réalisation.
La figure 13 est une vue en coupe qui représente un ensemble amovible selon un mode de réalisation comprenant un récipient de stockage, un organe distributeur, des éléments étanchéités et une veine aéraulique.
La figure 14 est une vue partielle agrandie en coupe selon la ligne XIV-XIV de l'ensemble amovible de la figure 9.

### Description des modes de réalisation

On va maintenant décrire plusieurs modes de réalisation d'un appareil diffuseur destiné à diffuser une substance à l'état vapeur dans l'air ambiant.

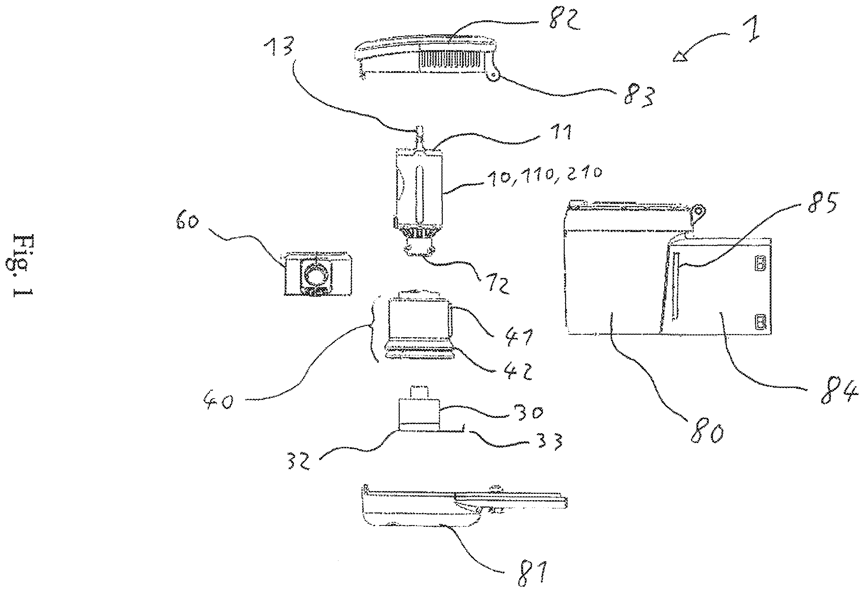

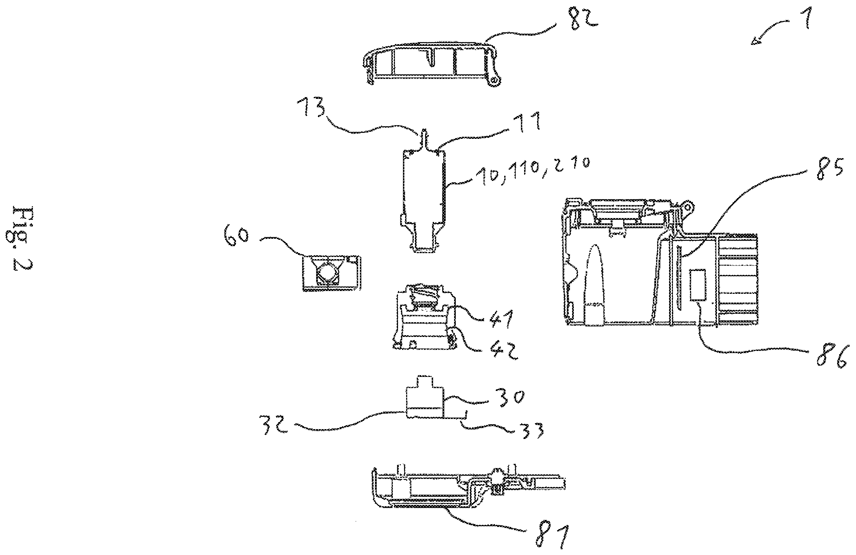

Les figures 1 et 2 illustrent un appareil diffuseur 1 constitué de plusieurs éléments représentés ici en perspective éclatée. Un récipient de stockage cylindrique 10, 110, 210 contient la substance liquide. Le récipient de stockage 10, 110, 210 comporte un corps cylindrique creux 17 fermé à son extrémité supérieure par un embout étanche 11 qui porte une languette de préhension 13 dans sa partie supérieure. Une partie inférieure du corps cylindrique creux 17 présente un orifice 12. L'orifice permet la communication de la substance liquide vers l'extérieur du récipient de stockage 10, 110, 210. L'appareil diffuseur 1 comporte une pièce de guidage d'air 40. La pièce de guidage d'air 40 comporte un guide flux 41 associé à une veine aéraulique 42 et sont destinés à être agencés autour de l'orifice 12 du récipient de stockage 10. Une chambre de brassage d'air 60 est destinée à être positionnée autour du récipient de stockage 10, 110, 210 et à être en communication avec le guide flux 41. La chambre de brassage 60 peut s'étendre sur tout ou partie de la hauteur du récipient de stockage 10, 110, 210. Un corps poreux 30 présente un ergot 31 faisant saillie sur sa face supérieure et est destiné à pénétrer l'orifice 12 ou à y être relié par un tuyau non représenté.

Le corps poreux 30 comprend dans sa partie inférieure un élément chauffant 32 permettant de chauffer le corps poreux 30 et une jonction électrique 33 destinée à se connecter à une carte électronique 85 agencée dans le boitier 80. Dans la position d'utilisation, la jonction électrique 33 est liée avec la carte électronique 85 et/ou une source d'énergie 86 et permet une alimentation en courant électrique et/ou en signaux de commande. Les signaux de commande peuvent être générés par la carte électronique 85, par exemple en mettant en œuvre un programme de commande définissant un cycle arrêt/marche de l'élément chauffant 32, des dates de début de cycle, dates de fin de cycle ou durées de cycle. La source d'énergie peut prendre différentes formes telles qu'une batterie, une liaison à un réseau électrique externe et/ou à panneau solaire.

Le boitier 80 comprend un couvercle 82 comportant des charnières 83, une pièce de fond 81 présentant une ouverture vers l'environnement extérieur et une pièce de fixation 84. Le couvercle 82 peut être basculé entre une position fermée et une position ouverte, qui permet de remplacer le récipient de stockage 10, 110, 210 lorsque celui-ci est vidé de sa substance à diffuser. En position fermée, le couvercle 82 peut également venir appuyer sur la languette de préhension 13 afin de maintenir le récipient de stockage 10, 110, 210 de manière stable dans le boitier 80.

Selon un mode de réalisation, l'appareil diffuseur 1 est réalisé en deux parties : une partie amovible comprenant au moins le récipient de stockage 10, 110, 210 pris seul ou en combinaison avec d'autres éléments, comme ce sera expliqué ci-dessous, et une partie fixe comprenant au moins le boitier 80 destiné à recevoir la partie amovible.

Le récipient de stockage 10 peut être réalisé de différentes façons.

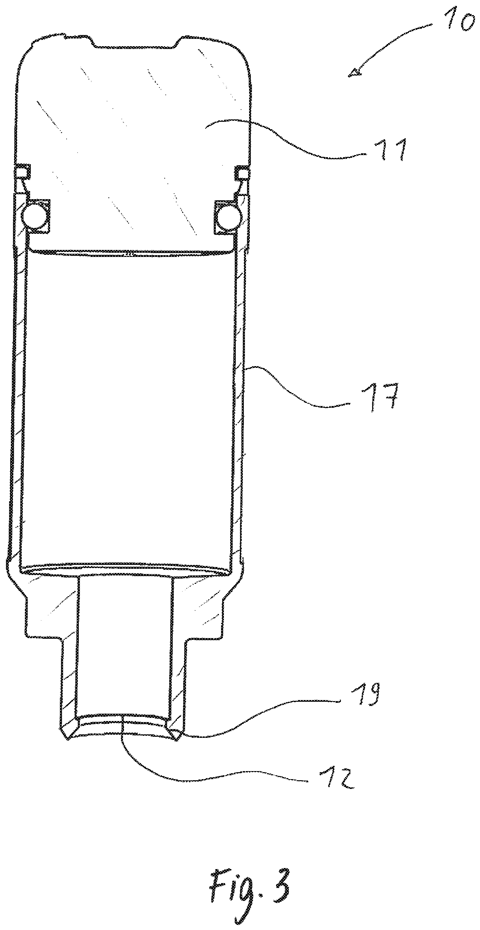

La figure 3 illustre un récipient de stockage 10 comporte un corps cylindrique creux 17 comprenant une partie supérieure fermée par un embout étanche 11 et une partie inférieure présentant un orifice 12 et une lèvre de maintien 19 agencée autour de l'orifice 12. L'embout étanche 11 permet de sceller le récipient de stockage 10 après remplissage. L'orifice 12 permet un écoulement de la substance contenue dans le récipient de stockage 10 vers le corps poreux 30.

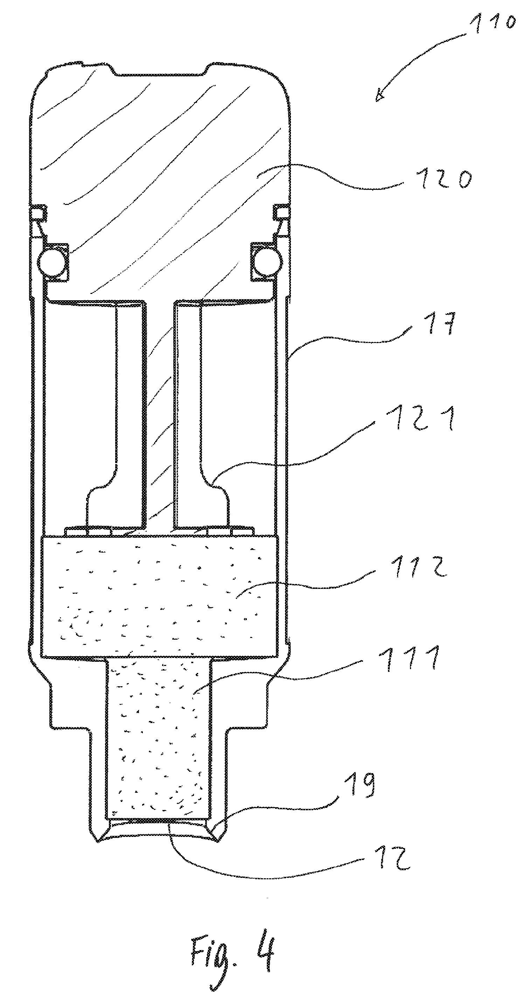

Selon un mode de réalisation représenté sur la figure 4, le récipient de stockage 110 contient deux blocs de mousses de rétention. Une première mousse 111 est agencée dans la zone d'embouchure du récipient de stockage 110 en retrait de l'orifice 12 et une deuxième mousse 112 est située dans le récipient de stockage 110 en contact avec la première mousse 111. Une tige 121 s'étend dans le récipient depuis l'embout étanche 120. La tige 121 présente par exemple avec une section en forme de croix. La tige 121 maintient en position les mousses de rétention. La tige 121 exerce une pression pour maintenir la deuxième mousse 112 en contact avec la première mousse 111 permettant ainsi de maintenir la première mousse 111 dans la zone d'embouchure du récipient de stockage 110. La première mousse 111 est située en retrait de l'orifice pour réduire les risques d'un contact accidentel avec des éléments extérieurs, contact qui pourrait conduire à polluer, endommager ou gaspiller de la substance liquide destinée à être diffusée. Une lèvre de maintien 19 est agencée autour de l'orifice 12. La lèvre de maintien 19 réduit la taille de l'orifice 12 et permet le maintien de l'organe de rétention alvéolaire 111 en retrait de l'orifice 12.

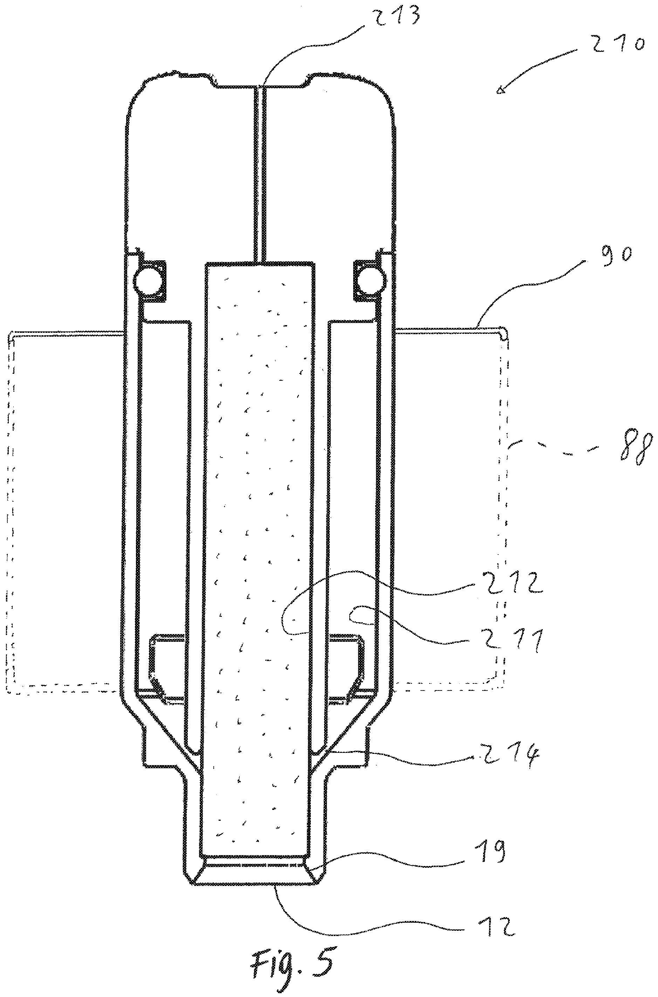

La figure 5 illustre un récipient de stockage 210 selon un autre mode de réalisation. Le récipient de stockage 210 contient un récipient de stockage extérieur 211 totalement clos si ce n'est à son extrémité en contact avec un récipient de stockage intérieur 212. Le réservoir intérieur 211 est surmonté d'un évent 213 en son extrémité supérieure. L'évent 213 permet l'équilibre des pressions entre l'air extérieur et l'intérieur du récipient de stockage intérieur 212. Le récipient de stockage intérieur 212 contient un organe de rétention alvéolaire, par exemple une éponge ou une mousse alvéolaire, qui est destinée à entrer en contact avec le corps poreux (non représenté ici) à travers l'orifice 12. Une lèvre de maintien 19 est agencée autour de l'orifice 12 et permet le maintien de l'organe de rétention alvéolaire 111 en retrait de l'orifice 12. Ainsi, à chaque goutte écoulée vers le corps poreux, la pression dans le récipient de stockage intérieur 212 s'équilibre par son évent 213, et entraine une baisse du niveau. Par vase communicant via la jonction 214 entre les deux récipients de stockage, le récipient de stockage extérieur 211 remplit le récipient de stockage intérieur 212, mais alors la dépression du récipient de stockage extérieur 211 augmente dans la partie du réservoir où il n'y a pas ou plus de liquide. Ainsi on équilibre le récipient de stockage intérieur 212 avec la dépression du récipient de stockage extérieur 211. Le récipient de stockage intérieur 212 peut toutefois toujours sortir de cet équilibre grâce à son évent 213 et la traction réalisée par le corps poreux de l'organe distributeur (non représenté ici). Pour que l'écoulement puisse s'effectuer normalement, lors de la mise en place du récipient de stockage 210 dans la partie fixe de l'appareil de diffusion, le récipient de stockage extérieur 211 est complètement rempli de la substance.

La figure 5 montre aussi qu'une collerette 90 peut être agencée faisant saillie autour du récipient de stockage 210 et adaptée pour recouvrir une ouverture d'une partie fixe de l'appareil diffuseur lors de son insertion. Dans ce cas, la partie fixe de l'appareil de diffusion ne comporte pas nécessairement de couvercle. La collerette 90 peut être positionnée au choix sur toute la longueur du récipient 210.

La partie fixe peut contenir tout ou partie d'une chambre de brassage, comme esquissé au chiffre 88. Selon un mode de réalisation, la collerette 90 constitue la paroi supérieure de la chambre de brassage 88 pour fermer celle-ci lorsque l'ensemble amovible est inséré en position de fonctionnement. Alternativement, la paroi supérieure 90 pourrait être solidaire de la partie fixe plutôt que de l'ensemble amovible. Dans ce cas, le récipient de stockage 210 vient fermer la chambre de brassage 88 en étant inséré dans une ouverture centrale de la paroi supérieur 90 dont le diamètre est égal au diamètre extérieur du récipient de stockage 210.

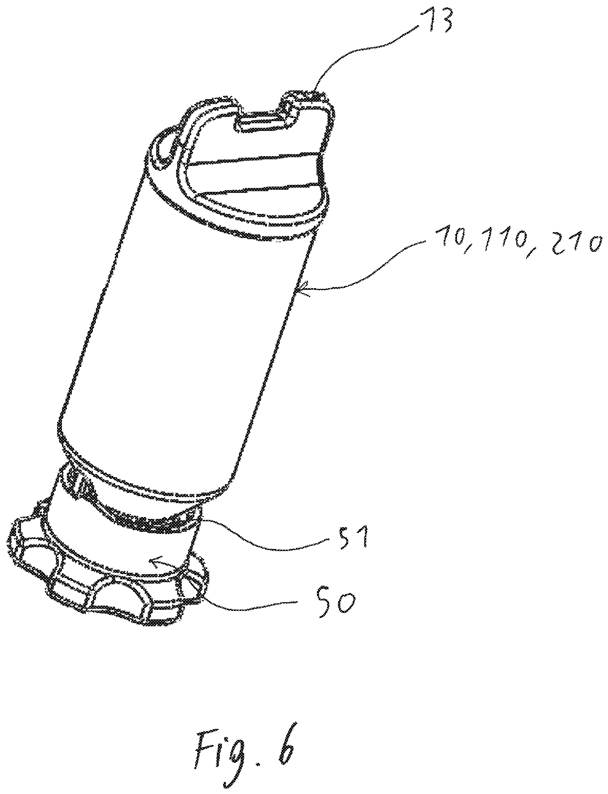

En référence à la figure 6, l'ouverture 12 du récipient de stockage 10, 110, 210 est équipée d'un bouchon de fermeture 50 afin d'empêcher la substance liquide contenue dans le récipient de stockage 10, 110, 210 de couler lorsque le récipient de stockage n'est pas utilisé et/ou lorsque qu'il est transporté. Ce bouchon 50 comporte une bague 51 supportant un joint torique. Le bouchon 50 peut être fixé par vissage ou par clipsage par exemple. L'utilisation d'un bouchon facilite le transport du récipient 10, 110, 210 et permet d'éviter les pertes de substances qui peuvent être onéreuses. L'étanchéité du bouchon permet d'éviter les pertes de substance même en cas de chute, de choc ou autre perturbation.

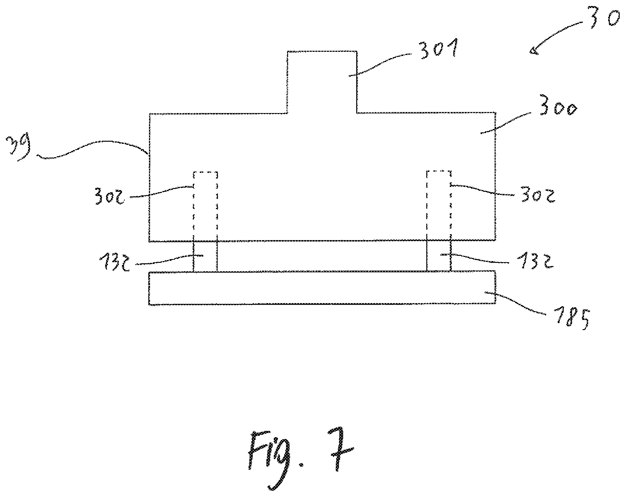

La figure 7 représente un mode de réalisation du corps poreux 30. Le corps poreux 30 comporte une partie principale 300 qui présente une forme cylindrique surmontée d'un ergot 301. Cet ergot permet de conduire la substance vers la partie principale 300 du corps poreux 30. Sur la face du corps poreux 30 opposée celle portant l'ergot 301, deux évidements 302 sont pratiqués afin d'accueillir chacun un élément chauffant 132 et/ou un capteur de température. Les éléments chauffants 132 sont des résistances électriques alimentées par un circuit électrique 185. Le capteur de température peut être une thermistance. La surface périphérique de la partie principale 300 forme une surface d'évaporation 39 sur laquelle débouchent des micro-canalisations formées par la porosité du corps poreux 30.

Le corps poreux 30 peut être intégré dans l'ensemble amovible avec le récipient 110 ou 210. Dans ce cas, l'ergot 301 sera inséré dans l'orifice 12 jusqu'à comprimer l'organe de rétention alvéolaire situé dans la zone d'embouchure du récipient de stockage 110, 210. Le corps poreux 30 peut aussi bien avoir une porosité uniforme que non-uniforme. Préférentiellement, la porosité ouverte est de 25% au cœur et de 45 % en surface. Ce sera alors un corps poreux dont la porosité ouverte, c'est-à-dire le volume des pores par unité de volume du corps poreux, augmente du cœur vers la surface d'évaporation. Cette structure favorise un étalement plus grand sur l'intégralité de la surface du corps poreux à la sortie des pores, et renforce l'intégrité mécanique du cœur poreux avec un cœur plus dense.

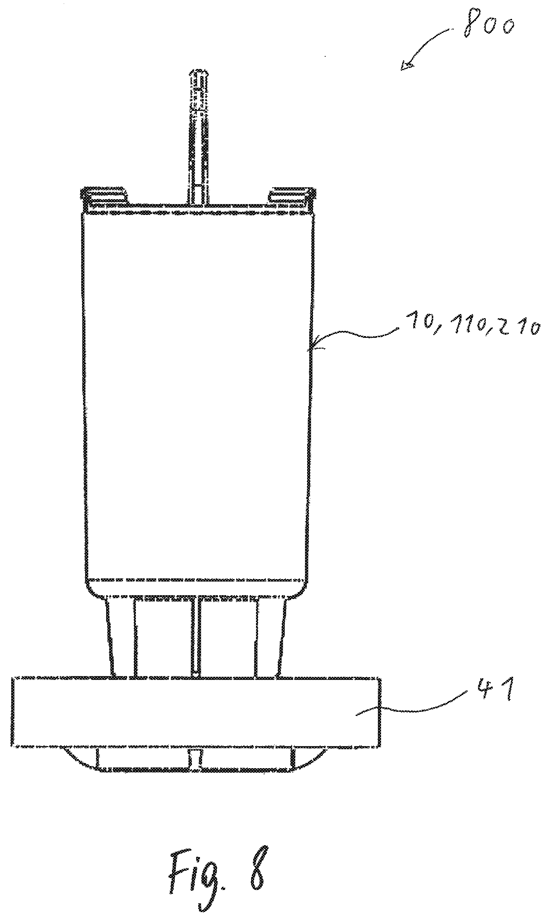

Le mode de réalisation de la figure 8 illustre un ensemble amovible 800 comprenant un récipient de stockage 10, 110, 210, et un guide flux d'air 41 situé en périphérie de l'orifice 12. Le guide flux d'air 41 est destiné guider un flux d'air dans une direction déterminée. Cet ensemble amovible 800 est destiné à être monté dans la partie fixe de l'appareil diffuseur comprenant notamment un générateur de flux d'air ainsi qu'un organe distributeur. Le flux d'air généré par le générateur de flux d'air en direction du guide flux d'air 41 va alors être guidé en direction de l'organe distributeur de la partie fixe de l'appareil de diffusion.

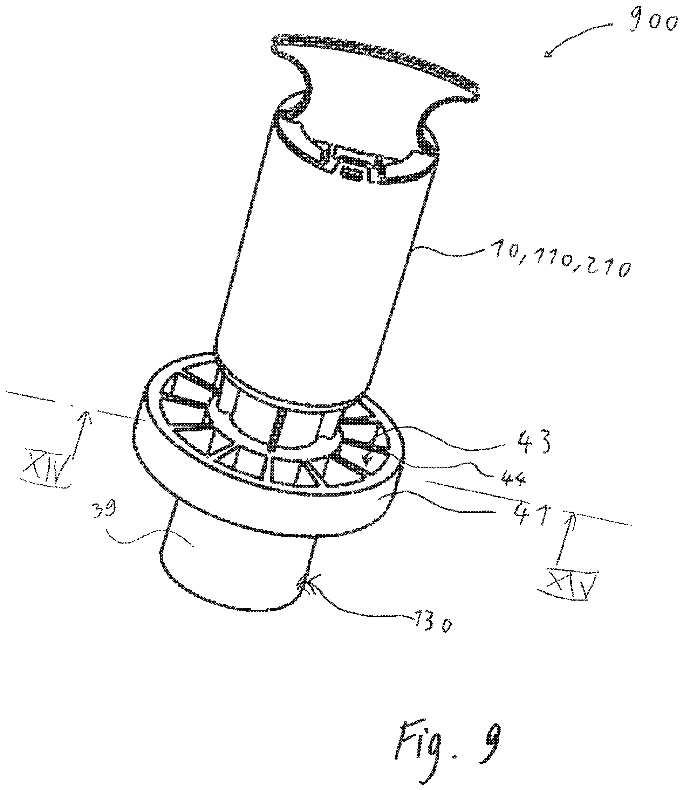

La figure 9 illustre un mode de réalisation d'un ensemble amovible 900 qui comporte en outre des éléments de la figure 8 un corps poreux 130 relié à l'orifice 12. Le corps poreux 130 est destiné à être relié à des organes chauffants situés dans la partie fixe de l'appareil diffuseur. La régulation de la diffusion de la substance peut être réalisée par contrôle de la température du corps poreux 130 avec un ou des organes chauffants intégrés dans la partie fixe de l'appareil diffuseur. Le guide flux d'air 41 comprend une pluralité d'ailettes 43 parallèles entres elles délimitant des canaux 44 permettant de guider le flux d'air en direction du corps poreux 130.

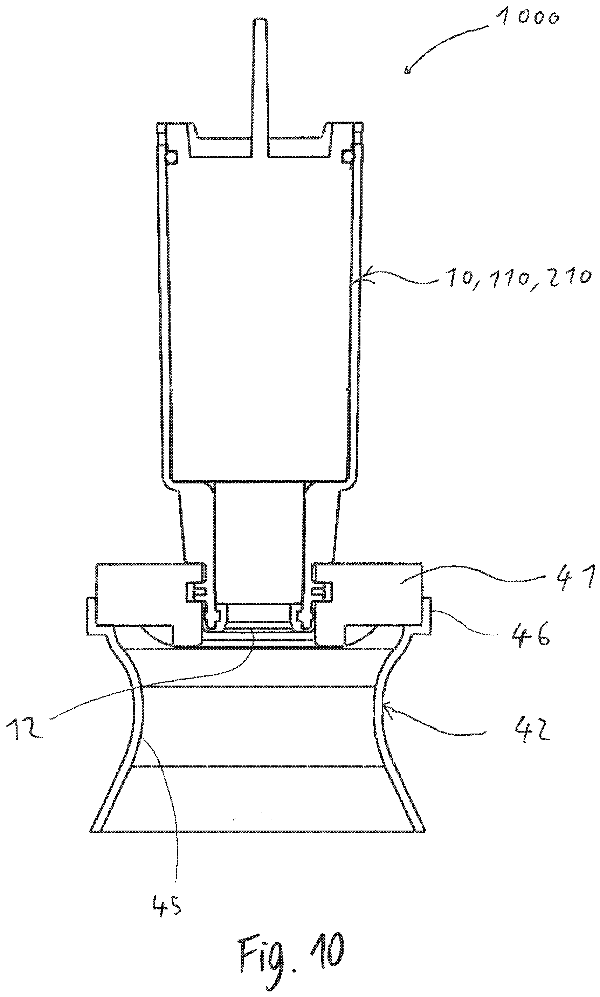

Le mode de réalisation de la figure 10 représente un ensemble amovible 1000 comprenant un récipient de stockage 10, 110, 210 associé avec un guide flux d'air 41 situé en périphérie de l'orifice 12 et une veine aéraulique tubulaire 42 fixée sur le guide flux d'air 41. La veine aéraulique 42 comporte un corps de révolution dont l'espace intérieur 45 présente une section convergente puis divergente. La plus petite section se trouve environ au niveau de la mi-longueur du corps poreux 30. Un rebord supérieur 46 de la veine aéraulique 42 vient attacher autour du guide flux 41, par exemple par encliquetage. La veine aéraulique 42 permet de guider le flux d'air sur le corps poreux 30 favorisant l'évaporation de la substance dans la direction souhaitée, à savoir vers le fond du boitier 80. Dans ce mode de réalisation, le corps poreux ainsi que le générateur de flux d'air peuvent se situer dans la partie fixe de l'appareil diffuseur.

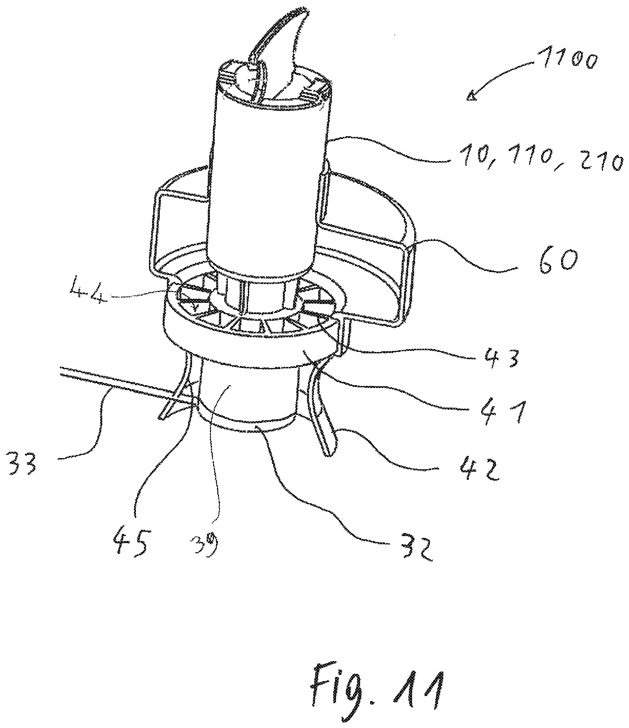

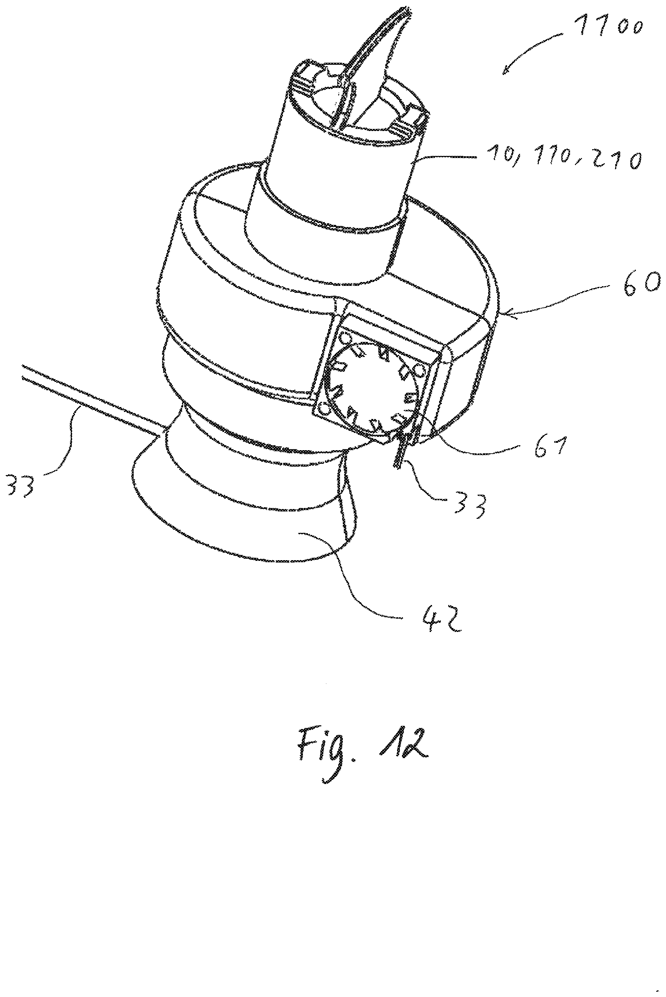

La figure 11 et la figure 12 illustrent un ensemble amovible 1100 comprenant un récipient 10, 110, 210, une chambre de brassage d'air 60 agencée autour du récipient de stockage 10,110,210, un guide flux d'air 41 comportant une pluralité d'ailettes 43 et une veine aéraulique 42. Les ailettes 43 sont parallèles à la direction axiale et disposées autour dudit récipient de stockage 10,110,210. La chambre de brassage d'air 60 peut comporter un ventilateur 61.

Le corps poreux 30 porte un élément chauffant électrique 32. Une jonction électrique 33 permet de relier l'élément chauffant électrique 32 à un circuit d'alimentation situé dans la partie fixe de l'appareil diffuseur.

En fonctionnement, l'élément chauffant électrique 32 va entrainer un réchauffement du corps poreux 30, une diminution de la viscosité de la substance liquide et par conséquence un écoulement de la substance liquide de l'embouchure du récipient de stockage en direction de l'organe distributeur. Le liquide affleure ainsi à l'extrémité des pores du corps poreux 30 par capillarité, dont l'ensemble constitue la surface d'évaporation ainsi située à la périphérie du corps poreux 30.

Lorsqu'un flux d'air pénètre la chambre de brassage d'air 60 il est alors dirigé via le guide flux d'air 41 et la veine aéraulique 42 tangentiellement sur la surface d'évaporation 39 pour entrainer une évaporation de la substance en surface et son transport à l'état vapeur dans la direction du flux d'air.

La force capillaire est provoquée par la nature de la surface, qui est constituée de canaux ou de pores suffisamment étroits pour générer une traction capillaire. Il faut que la force de traction et de rétention capillaire permette un affleurement du liquide à l'extrémité des pores de la surface d'évaporation 39 ; néanmoins, cet affleurement doit s'effectuer sans permettre un étalement incontrôlé sur la surface d'évaporation 39 via les forces dues aux champs de gravité (attraction terrestre et pression hydrostatique de la colonne de liquide potentiellement présente) ou aux forces d'attraction statiques générées par les interactions entre la solution et le reste de la surface du corps poreux 30.

Cette traction capillaire n'existe que par renouvellement du bloc volume final (la section cylindrique de liquide à l'extrémité du pore). Le renouvellement de ce volume est effectué par l'évaporation et est régi par l'équilibre des concentrations des molécules liquides et gazeuses à l'interface liquide et gaz selon une grandeur propre à chaque solution et dépendante principalement de la température (à pression atmosphérique), à savoir, la pression de vapeur saturante. L'augmentation de la température de la solution à évaporer entraine une augmentation de la pression de vapeur saturante, donc le déplacement de l'équilibre des concentrations de molécules liquides et gazeuses à l'interface vers les molécules gazeuses : il y a évaporation jusqu'au nouvel équilibre. Si la phase gazeuse est mobile, l'équilibre n'est jamais atteint et l'évaporation se poursuit jusqu'à épuisement de la phase liquide. Plus la phase gazeuse est mobile (et tend plus vite à évacuer les molécules en phase gaz) plus l'évaporation est rapide.

En position d'utilisation, le ventilateur 61 est relié par jonction électronique à la carte électronique 85 et un générateur d'énergie électrique 86. Le ventilateur 61 génère un flux d'air au sein de la chambre de brassage 60 dans une direction perpendiculaire aux ailettes 43 du guide flux. Le flux d'air subit une perturbation et un changement de direction via les ailettes 43 du guide flux 60 et pénètre la veine aéraulique 42, le flux d'air est alors dirigé vers le corps poreux 30 comportant l'élément chauffant 32. Comme expliqué précédemment, la chauffe du corps poreux 30 va diminuer la viscosité de la substance et avec le flux d'air cela va permettre l'évaporation de la substance vers l'environnement extérieur. Dans une variante du mode de réalisation, le ventilateur 61 est monté dans la partie fixe de l'appareil diffuseur et un orifice est présent dans la chambre de brassage 60 permettant l'intégration du ventilateur 61 lorsque l'ensemble amovible est monté dans la partie fixe de l'appareil diffuseur.

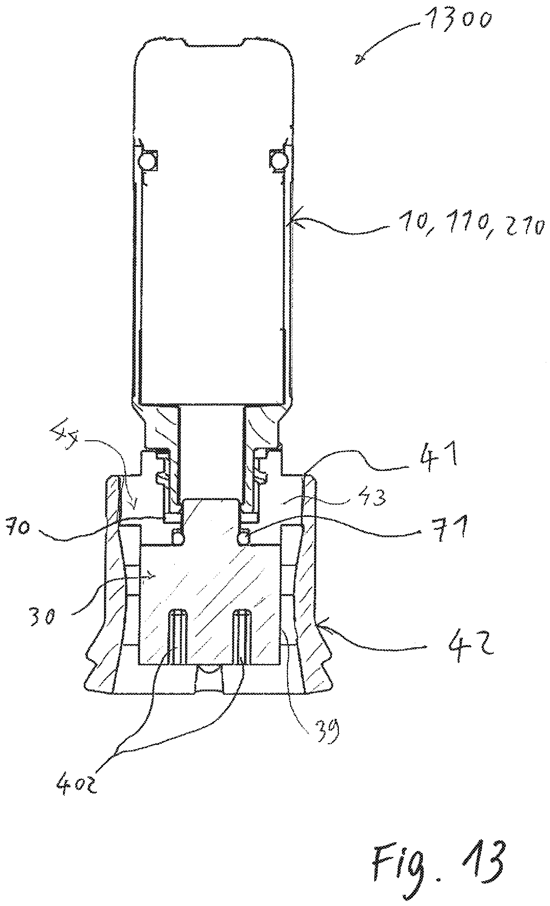

La figure 13 représente un ensemble amovible 1300 comprenant un récipient 10, 110, 210, un guide flux 41 et une veine aéraulique 42 situé tout autour du corps poreux. Le guide flux 41 et la veine aéraulique 42 peuvent être formés d'une seule pièce. Une double d'étanchéité est agencée entre le récipient 10, 110, 210, la veine aéraulique 42 et le corps poreux 30. Un élément d'étanchéité 70 est situé entre le récipient 10, 110, 210 et la veine aéraulique 42 et un élément d'étanchéité 71 est situé entre la veine aéraulique 42 et le corps poreux 30. Le corps poreux 30 comprend deux évidements 402 pratiqués afin d'accueillir chacun un élément chauffant132 et/ou un capteur de température. Dans ce mode de réalisation, en position d'utilisation les deux évidements de l'ensemble amovible viennent s'emboiter à deux éléments chauffants 132 de la partie fixe de l'appareil diffuseur.

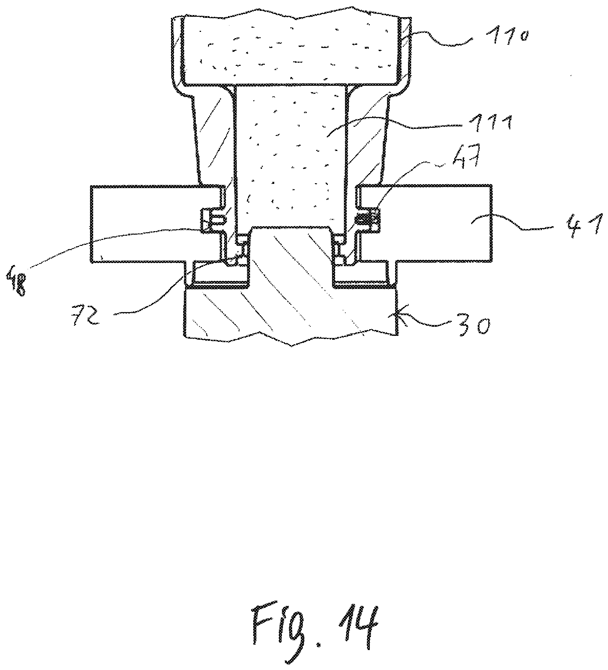

La figure 14 illustre une coupe de l'ensemble amovible 900 comportant un récipient de stockage 110, un guide flux d'air 41 et un corps poreux 30. Le corps poreux 30 est inséré dans l'orifice 12 du récipient 110 jusqu'à venir comprimer l'organe de rétention alvéolaire 111 en formant alors une jonction de contact. Le guide flux 41 est agencé autour du récipient de stockage 110, au niveau de la jonction entre le récipient de stockage 110 et le corps poreux 30. Un joint d'étanchéité torique 72 est monté au niveau de la jonction entre le récipient de stockage 110 et le corps poreux 30. Un autre joint d'étanchéité 47 peut être logé dans une rainure 48 du guide flux 41. Les étanchéités permettent en position d'utilisation de contraindre l'écoulement du liquide en direction du corps poreux 30 sans aucune fuite annexe de substance. Les étanchéités peuvent être par exemple simple, double, radiale et/ou compressive.

Bien que l'invention ait été décrite en liaison avec plusieurs modes de réalisation particuliers, il est bien évident qu'elle n'y est nullement limitée.

L'usage du verbe « comporter », « comprendre » ou « inclure » et de ses formes conjuguées n'exclut pas la présence d'autres éléments ou d'autres étapes que ceux énoncés dans une revendication.

Dans les revendications, tout signe de référence entre parenthèses ne saurait être interprété comme une limitation de la revendication.

### Exemples :

Plusieurs modes de réalisation de l'appareil diffuseur 1 ont été fabriqués et testés.

### Expérience 1 :

La molécule active utilisée est le (7E,9Z)-Dodeca-7,9-dienylacetate ayant un point éclair de 137 °C, une Masse volumique (20°C) de 0,903 g/cm3, une viscosité de 8mPa.s à 23°C et une pression de vapeur saturante de 0,15 Pa à 15 °C.

La composition étudiée comprend :
- 99,5 % en poids de (7E,9Z)-Dodeca-7,9-dienylacetate, et
- 0,5 % en poids de BHT (hydroxytoluène butylé).

Cette composition est connue sous le nom commercial de Rak^{®} 2 New et sera utilisée dans le récipient de stockage pour cette expérience.

Trois systèmes ont été étudiés :
Le corps poreux est une mèche de céramique en alumine frittée présentant une taille de pores de 100 nm et une porosité uniforme de 40 %

### Système 1 :

La surface d'évaporation de la mèche est de 7,9 cm², la mèche présente un diamètre de 2,3 cm (centimètre) par une hauteur de 1.2 cm. La vitesse moyenne du flux d'air autour de la surface est de 0,2 m/s. La température extérieure est de 25 °C.

### Système 2 :

La surface d'évaporation de la mèche est de 15,5 cm², la mèche présente un diamètre de 2,6 cm (centimètre) par une hauteur de 1,9 cm. La vitesse moyenne du flux d'air autour de la surface est de 0,45 m/s. La température extérieure est de 25 °C.

### Système 3 :

La surface d'évaporation de la mèche est de 22,8 cm², la mèche présente un diamètre de 2,8 cm (centimètre) par une hauteur de 2,6 cm. La vitesse moyenne du flux d'air autour de la surface est de 0,50 m/s. La température extérieure est de 25 °C.

| Température de contrôle | Ambiant | 52°C | 58°C |
|---|---|---|---|
| Evaporation RAK 2 (mg/h) sur Système 1 | 0 | 1,9 | 3,8 |

| Système | Système 1 | Système 2 | Système 3 |
|---|---|---|---|
| Surface d'évaporation (cm²) | 7,9 | 15,5 | 22,8 |
| Evaporation RAK 2 (mg/h) à 58°C | 3.8 | 8.9 | 12.2 |
| Température seuil de non évaporation °C | 33 | 33 | 33 |

Les mesures sont réalisées selon un protocole de perte de masse relative du système, en considérant (par constat visuel) que toute perte de masse relève d'une évaporation puis expulsion des molécules hors du système.

### Expérience 2 :

La molécule utilisée est l'Isopropyle Myristate ayant un point éclair de 160°C, une masse volumique de 0,855 g/cm3 à 20°C, une viscosité dynamique de 5,58 mPa.s à 20°C et une pression de vapeur saturante de 100 Pa à 20°C.

### Système 1 :

La surface d'évaporation de la mèche est de 7,9 cm², la mèche présente un diamètre de 2,3 cm (centimètre) par une hauteur de 1.2 cm. La vitesse moyenne du flux d'air autour de la surface est de 0,2 m/s. La température extérieure est de 25 °C.

### Système 2 :

La surface d'évaporation de la mèche est de 15,5 cm², la mèche présente un diamètre de 2,6 cm (centimètre) par une hauteur de 1,9 cm. La vitesse moyenne du flux d'air autour de la surface est de 0,2 m/s. La température extérieure est de 25 °C.

### Système 3 :

La surface d'évaporation de la mèche est de 15,5 cm², la mèche présente un diamètre de 2,6 cm (centimètre) par une hauteur de 1,9 cm. La vitesse moyenne du flux d'air autour de la surface est de 0,45 m/s. La température extérieure est de 25 °C.

| Température de contrôle | Ambiant | 50°C | 60°C | 70°C |
|---|---|---|---|---|
| Evaporation Isopropyle Myristate (mg/h) sur Système 1 | 0 | 8,9 | 23,1 | 45,5 |

| Système | Système 1 | Système 2 | Système 3 | |
|---|---|---|---|---|
| Surface d'évaporation (cm2) | 7,9 | 15,5 | 15.5 | |
| Evaporation Isopropyle Myristate (mg/h) à 55°C | 19.5 | 21.1 | 44.4 | |
| Température seuil de non évaporation °C | 36 | 36 | 36 | |

Les mesures sont réalisées selon un protocole de perte de masse relative du système, en considérant (par constat visuel) que toute perte de masse relève d'une évaporation puis expulsion des molécules hors du système.

## Revendications

1. 'Ensemble amovible pour un appareil diffuseur (1), l'appareil diffuseur (1) étant destiné à diffuser une substance à l'état vapeur dans l'air ambiant, l'ensemble amovible comprenant :
- un récipient de stockage (10), ledit récipient de stockage (10, 110, 210) comprenant un orifice (12),
ledit récipient de stockage (10, 110, 210) contenant une substance liquide et un organe distributeur (30, 130) positionné en sortie dudit orifice (12) et relié audit orifice (12) pour évaporer la substance dans l'air ambiant,
ladite substance liquide comportant au moins un composé sélectionné parmi les molécules sémiochimiques, les phéromones, les allomones, les kairomones, les synomones et les parfums d'origine naturelle ou synthétique,
ledit organe distributeur (30, 130) comprenant un corps poreux (30, 130) présentant une surface d'évaporation (39) située à l'extérieur dudit récipient de stockage (10, 110, 210), **caractérisé en ce que**
l'orifice du récipient relié à l'organe distributeur étant orienté vers le bas, c'est-à-dire dans la direction de l'accélération de la pesanteur, lorsque l'appareil est dans une position d'utilisation, et ladite substance présentant une viscosité variable en fonction de la température, ladite viscosité et la tension superficielle de la substance liquide sont telles que l'accélération de pesanteur s'exerçant depuis l'orifice (12) en direction du corps poreux (30, 130) et constituant une force motrice susceptible de générer un écoulement depuis le récipient de stockage vers ladite surface d'évaporation (39), ladite substance ne s'écoule pas à travers ledit corps poreux (30, 130) à toute température ambiante inférieure à une première température, la première température étant supérieure à 0°C et que ladite substance s'écoule à travers ledit corps poreux (30, 130) à une deuxième température supérieure à ladite première température.

2. Ensemble amovible selon la revendication 1, **caractérisé en ce que** la première température est comprise entre 1 et 50°C.

3. Ensemble amovible selon la revendication 1, **caractérisé en ce que** ledit ensemble amovible comporte au moins un organe de chauffage (32) permettant de chauffer ledit corps poreux (30, 130) et un contact électrique (33) associé à l'organe de chauffage et destiné à réaliser une liaison électrique avec une partie fixe de l'appareil diffuseur (1).

4. Ensemble amovible selon la revendication 3, comportant en outre au moins un élément électronique de régulation configuré pour réguler l'organe de chauffage (32).

5. Ensemble amovible selon l'une quelconque des revendications 1 à 4, **caractérisé en ce que** le corps poreux (30, 130) comporte des pores présentant un diamètre compris entre 0.01 et 10 µm.

6. Ensemble amovible selon l'une des revendications 1 à 5, **caractérisé en ce que** ledit ensemble amovible comprend un guide flux d'air (41) situé en périphérie dudit orifice (12), ledit guide flux d'air (41) étant destiné à guider un flux d'air dans une direction tangentielle à la surface d'évaporation (39), dans lequel ledit ensemble amovible comprend des éléments étanchéités pour réaliser des liaisons étanches entre le récipient de stockage (10) et ledit guide flux d'air (41) et entre le guide flux d'air (41) et ledit corps poreux (30, 130).

7. Ensemble amovible selon la revendication 6 **caractérisé en ce que** ledit récipient de stockage (10) présente une forme allongée selon une direction axiale, ledit orifice (12) étant situé à une extrémité axiale du récipient de stockage (10), la surface d'évaporation (39) du corps poreux (30, 130) étant parallèle à la direction axiale, ledit guide flux d'air (41) comportant une pluralité d'ailettes 43 parallèles à la direction axiale et disposées autour dudit récipient de stockage (10).

8. Ensemble amovible selon la revendication 6 ou 7, **caractérisé en ce que** ledit ensemble amovible comprend une veine aéraulique (42) tubulaire s'étendant autour du corps poreux (30, 130), ladite veine aéraulique (42) permettant de guider le flux d'air sur ledit corps poreux (30, 130).

9. Ensemble amovible selon l'une des revendications 1 à 8, **caractérisé en ce que** ledit ensemble amovible comprend tout ou partie d'une chambre de brassage d'air (60), ladite chambre de brassage d'air (60) s'étendant tout autour dudit récipient de stockage (10).

10. Ensemble amovible selon la revendication 9, **caractérisé en ce que** ladite chambre de brassage d'air (60) comprend une ouverture permettant la mise en relation avec un ventilateur.

11. Ensemble amovible selon la revendication 10, **caractérisé en ce que** ladite chambre de brassage d'air (60) comprend un ventilateur (61) et une jonction électrique 33, ledit ventilateur (61) étant positionné de manière à générer un flux d'air au sein de ladite chambre de brassage d'air (60), ladite jonction électrique (33) étant destinée à s'associer à un connecteur électrique au sein d'une partie fixe de l'appareil diffuseur (1) pour alimenter le ventilateur (61).

12. Ensemble amovible selon l'une des revendications 9 à 11 prise en combinaison avec la revendication 5, dans lequel ledit guide flux d'air (41) présente une pluralité de canaux (44) débouchant sur la chambre de brassage d'air (60) et orientés dans la direction tangentielle à la surface d'évaporation (39).

13. Ensemble amovible selon l'une des revendications 1 à 12, **caractérisé en ce que** ledit ensemble amovible comprend une collerette (90) faisant saillie autour du récipient de stockage (10) et adaptée pour recouvrir une ouverture d'une partie fixe de l'appareil diffuseur (1), ladite ouverture étant destinée à l'insertion dudit ensemble amovible dans la partie fixe de l'appareil diffuseur (1).

14. Ensemble amovible selon l'une quelconque des revendications 1 à 13, **caractérisé en ce que** le récipient de stockage (10) comporte un évent de pressurisation (213) mettant en communication l'espace intérieur du récipient de stockage (212) avec l'air ambiant.

15. Ensemble amovible selon l'une des revendications 1 à 14, comportant en outre un bouchon (50) disposé pour boucher ledit orifice (12) de manière étanche durant le transport dudit ensemble amovible.

16. Appareil de diffusion **caractérisé en ce que** ledit appareil de diffusion (1) comprend :
- ledit ensemble amovible (900, 1000, 1100, 1300) selon l'une quelconque des revendications précédentes, et
- une partie fixe comportant un boîtier (80) qui définit un logement dans lequel ledit ensemble amovible est inséré dans une position de fonctionnement permettant la diffusion de la substance à l'état vapeur dans l'air ambiant.

## Patentansprüche

1. Abnehmbare Baugruppe für ein Diffusionsgerät (1), wobei das Diffusionsgerät (1) dazu bestimmt ist, eine Substanz im Dampfzustand in der Umgebungsluft zu verteilen, wobei die abnehmbare Baugruppe Folgendes umfasst:
- einen Vorratsbehälter (10), wobei der Vorratsbehälter (10, 110, 210) eine Öffnung (12) umfasst,
wobei der Vorratsbehälter (10, 110, 210) eine flüssige Substanz und ein Abgabeelement (30, 130) enthält, das am Austritt der Öffnung (12) angeordnet und mit der Öffnung (12) verbunden ist, um die Substanz in der Umgebungsluft zu verdampfen,
wobei die flüssige Substanz mindestens eine Verbindung umfasst, die aus semiochemischen Molekülen, Pheromonen, Allomonen, Kairomonen, Synomonen und Duftstoffen natürlichen oder synthetischen Ursprungs ausgewählt ist,
wobei das Abgabeelement (30, 130) einen porösen Körper (30, 130) umfasst, der eine außerhalb des Vorratsbehälters (10, 110, 210) gelegene Verdampfungsfläche (39) aufweist, **dadurch gekennzeichnet, dass**
die mit dem Abgabeelement verbundene Öffnung des Behälters nach unten, das heißt in Richtung der Schwerebeschleunigung, gerichtet ist, wenn sich das Gerät in einer Gebrauchsstellung befindet, und die Substanz eine in Abhängigkeit von der Temperatur veränderliche Viskosität aufweist, wobei die Viskosität und die Oberflächenspannung der flüssigen Substanz derart sind, dass bei einer von der Öffnung (12) in Richtung des porösen Körpers (30, 130) wirkenden Schwerebeschleunigung, die eine treibende Kraft darstellt, welche eine Strömung aus dem Vorratsbehälter zur Verdampfungsfläche (39) erzeugen kann, die Substanz bei keiner Umgebungstemperatur unterhalb einer ersten Temperatur durch den porösen Körper (30, 130) strömt, wobei die erste Temperatur höher als 0°C ist, und dass die Substanz bei einer zweiten Temperatur, die höher als die erste Temperatur ist, durch den porösen Körper (30, 130) strömt.

2. Abnehmbare Baugruppe nach Anspruch 1, **dadurch gekennzeichnet, dass** die erste Temperatur zwischen 1 und 50°C liegt.

3. Abnehmbare Baugruppe nach Anspruch 1, **dadurch gekennzeichnet, dass** die abnehmbare Baugruppe mindestens ein Heizelement (32) zum Erwärmen des porösen Körpers (30, 130) und einen dem Heizelement zugeordneten elektrischen Kontakt (33) umfasst, der dazu bestimmt ist, eine elektrische Verbindung mit einem festen Teil des Diffusionsgeräts (1) herzustellen.

4. Abnehmbare Baugruppe nach Anspruch 3, ferner umfassend mindestens ein elektronisches Regelelement, das zum Regeln des Heizelements (32) eingerichtet ist.

5. Abnehmbare Baugruppe nach einem der Ansprüche 1 bis 4, **dadurch gekennzeichnet, dass** der poröse Körper (30, 130) Poren aufweist, deren Durchmesser zwischen 0,01 und 10 µm liegt.

6. Abnehmbare Baugruppe nach einem der Ansprüche 1 bis 5, **dadurch gekennzeichnet, dass** die abnehmbare Baugruppe eine im Umfangsbereich der Öffnung (12) angeordnete Luftstromführung (41) umfasst, wobei die Luftstromführung (41) dazu bestimmt ist, einen Luftstrom in einer zur Verdampfungsfläche (39) tangentialen Richtung zu führen, wobei die abnehmbare Baugruppe Dichtungselemente zur Herstellung dichter Verbindungen zwischen dem Vorratsbehälter (10) und der Luftstromführung (41) sowie zwischen der Luftstromführung (41) und dem porösen Körper (30, 130) umfasst.

7. Abnehmbare Baugruppe nach Anspruch 6, **dadurch gekennzeichnet, dass** der Vorratsbehälter (10) eine in einer axialen Richtung längliche Form aufweist, wobei die Öffnung (12) an einem axialen Ende des Vorratsbehälters (10) angeordnet ist, die Verdampfungsfläche (39) des porösen Körpers (30, 130) parallel zur axialen Richtung verläuft und die Luftstromführung (41) eine Vielzahl von Lamellen 43 umfasst, die parallel zur axialen Richtung verlaufen und um den Vorratsbehälter (10) herum angeordnet sind.

8. Abnehmbare Baugruppe nach Anspruch 6 oder 7, **dadurch gekennzeichnet, dass** die abnehmbare Baugruppe einen rohrförmigen Luftkanal (42) umfasst, der sich um den porösen Körper (30, 130) herum erstreckt, wobei der Luftkanal (42) es ermöglicht, den Luftstrom über den porösen Körper (30, 130) zu führen.

9. Abnehmbare Baugruppe nach einem der Ansprüche 1 bis 8, **dadurch gekennzeichnet, dass** die abnehmbare Baugruppe eine Luftmischkammer (60) ganz oder teilweise umfasst, wobei sich die Luftmischkammer (60) rings um den Vorratsbehälter (10) erstreckt.

10. Abnehmbare Baugruppe nach Anspruch 9, **dadurch gekennzeichnet, dass** die Luftmischkammer (60) eine Öffnung umfasst, die eine Verbindung mit einem Ventilator ermöglicht.

11. Abnehmbare Baugruppe nach Anspruch 10, **dadurch gekennzeichnet, dass** die Luftmischkammer (60) einen Ventilator (61) und einen elektrischen Anschluss 33 umfasst, wobei der Ventilator (61) so angeordnet ist, dass er innerhalb der Luftmischkammer (60) einen Luftstrom erzeugt, und der elektrische Anschluss (33) dazu bestimmt ist, mit einem elektrischen Verbinder innerhalb eines festen Teils des Diffusionsgeräts (1) verbunden zu werden, um den Ventilator (61) mit Strom zu versorgen.

12. Abnehmbare Baugruppe nach einem der Ansprüche 9 bis 11 in Kombination mit Anspruch 5, wobei die Luftstromführung (41) eine Vielzahl von Kanälen (44) aufweist, die in die Luftmischkammer (60) münden und in der zur Verdampfungsfläche (39) tangentialen Richtung ausgerichtet sind.

13. Abnehmbare Baugruppe nach einem der Ansprüche 1 bis 12, **dadurch gekennzeichnet, dass** die abnehmbare Baugruppe einen Kragen (90) umfasst, der um den Vorratsbehälter (10) herum vorsteht und dazu ausgebildet ist, eine Öffnung eines festen Teils des Diffusionsgeräts (1) zu überdecken, wobei die Öffnung zum Einsetzen der abnehmbaren Baugruppe in den festen Teil des Diffusionsgeräts (1) bestimmt ist.

14. Abnehmbare Baugruppe nach einem der Ansprüche 1 bis 13, **dadurch gekennzeichnet, dass** der Vorratsbehälter (10) eine Druckausgleichsöffnung (213) aufweist, die den Innenraum des Vorratsbehälters (212) mit der Umgebungsluft verbindet.

15. Abnehmbare Baugruppe nach einem der Ansprüche 1 bis 14, ferner umfassend einen Verschlussstopfen (50), der dazu angeordnet ist, die Öffnung (12) während des Transports der abnehmbaren Baugruppe dicht zu verschließen.

16. Diffusionsgerät, **dadurch gekennzeichnet, dass** das Diffusionsgerät (1) Folgendes umfasst:
- die abnehmbare Baugruppe (900, 1000, 1100, 1300) nach einem der vorhergehenden Ansprüche; und
- einen festen Teil, der ein Gehäuse (80) umfasst, das eine Aufnahme definiert, in die die abnehmbare Baugruppe in einer Betriebsstellung eingesetzt ist, welche die Verteilung der Substanz im Dampfzustand in der Umgebungsluft ermöglicht.

## Claims

1. A removable assembly for a diffusing apparatus (1), the diffusing apparatus (1) being intended to diffuse a substance in the vapor state into the surrounding air, the removable assembly including:
- a storage container (10), said storage container (10, 110, 210) including an orifice (12),
said storage container (10, 110, 210) containing a liquid substance and a distributor member (30, 130) positioned at the outlet of said orifice (12) and connected to said orifice (12) to evaporate the substance into the surrounding air,
said liquid substance including at least one compound selected from semiochemical molecules, pheromones, allomones, kairomones, synomones and fragrances of natural or synthetic origin,
said distributor member (30, 130) including a porous body (30, 130) having an evaporation surface (39) situated outside said storage container (10, 110, 210),
**characterized in that** the orifice of the container connected to the distributor member being oriented downward, that is to say in the direction of the acceleration due to gravity, when the apparatus is in a position of use, and
said substance having a viscosity varying as a function of temperature, said viscosity and the surface tension of the liquid substance are such that the acceleration due to gravity being exerted from the orifice (12) in the direction of the porous body (30, 130) and constituting a motive force liable to generate a flow from the storage container toward said evaporation surface (39), said substance does not flow through said porous body (30, 130) at any ambient temperature below a first temperature, the first temperature being greater than 0°C, and that said substance flows through said porous body (30, 130) at a second temperature higher than said first temperature.

2. The removable assembly as claimed in claim 1, **characterized in that** the first temperature is between 1 and 50°C.

3. The removable assembly as claimed in claim 1, **characterized in that** said removable assembly includes at least one heating member (32) enabling heating of said porous body (30, 130) and an electric contact (33) associated with the heating member and intended to make an electric connection with a fixed part of the diffusing apparatus (1).

4. The removable assembly as claimed in claim 3, further including at least one electronic regulator element configured to regulate the heating member (32).

5. The removable assembly as claimed in any one of claims 1 to 4, **characterized in that** the porous body (30, 130) includes pores having a diameter between 0.01 and 10 µm.

6. The removable assembly as claimed in any one of claims 1 to 5, **characterized in that** said removable assembly includes an air flow guide (41) situated at the periphery of said orifice (12), said air flow guide (41) being intended to guide a flow of air in a direction tangential to the evaporation surface (39), in which said removable assembly includes sealing elements for producing sealed connections between the storage container (10) and said air flow guide (41) and between the air flow guide (41) and said porous body (30, 130).

7. The removable assembly as claimed in claim 6 **characterized in that** said storage container (10) has an elongate shape in an axial direction, said orifice (12) being situated at an axial end of the storage container (10), the evaporation surface (39) of the porous body (30, 130) being parallel to the axial direction, said air flow guide (41) including a plurality of fins 43 parallel to the axial direction and disposed around said storage container (10).

8. The removable assembly as claimed in claim 6 or 7, **characterized in that** said removable assembly includes a tubular chute (42) extending around the porous body (30, 130), said chute (42) enabling the flow of air to be guided onto said porous body (30, 130).

9. The removable assembly as claimed in any one of claims 1 to 8, **characterized in that** said removable assembly includes all or part of an air mixer chamber (60), said air mixer chamber (60) extending all around said storage container (10).

10. The removable assembly as claimed in claim 9, **characterized in that** said air mixer chamber (60) includes an opening allowing connection to a fan.

11. The removable assembly as claimed in claim 10, **characterized in that** said air mixer chamber (60) includes a fan (61) and an electric connector (33), said fan (61) being positioned in such a manner as to generate a flow of air in said air mixer chamber (60), said electric connector (33) being intended to be associated with an electric connector in a fixed part of the diffusing apparatus (1) to supply power to the fan (61).

12. The removable assembly as claimed in any one of claims 9 to 11 in combination with claim 5, in which said air flow guide (41) includes a plurality of channels (44) opening into the air mixer chamber (60) and oriented in a direction tangential to the evaporation surface (39).

13. The removable assembly as claimed in any one of claims 1 to 12, **characterized in that** said removable assembly includes a flange (90) projecting around the storage container (10) and adapted to cover an opening in a fixed part of the diffusing apparatus (1), said opening being intended for the insertion of said removable assembly in the fixed part of the diffusing apparatus (1).

14. The removable assembly as claimed in any one of claims 1 to 13, **characterized in that** the storage container (10) includes a pressurization vent (213) establishing communication between the interior space of the storage container (212) and the surrounding air.

15. The removable assembly as claimed in any one of claims 1 to 14, further including a stopper (50) disposed to block said orifice (12) in sealed manner during transportation of said removable assembly.

16. Diffusing apparatus **characterized in that** said diffusing apparatus (1) includes:
- said removable assembly (900, 1000, 1100, 1300) as claimed in any one of the preceding claims, and
- a fixed part including a casing (80) that defines a housing in which said removable assembly is inserted in an operating position enabling diffusion of the substance in the vapor state into the surrounding air.
